# EUROPEAN PATENT APPLICATION

(11) **EP 4 379 378 A1**
(43) Date of publication of application: **05.06.2024**
(21) Application number: 22210259.2
(22) Date of filing: 29.11.2022
(51) Int. Cl.: G01N 33/543, C12Q 1/6869

(54) **SELECTIVE-AREA COATED PROTEIN RESISTANT SURFACE**

(71) Applicant: Gnothis Holding AG, 6330 Cham (CH)
(72) Inventor: WIND, Samuel (Shalom) J., White Plains, NY, 10605 (US); EDMAN, Lars, 61992 Trosa (SE); REDIN, David, 61992 Trosa (SE); LUNDBERG, Oscar, 61992 Trosa (SE)
(74) Representative: Weickmann & Weickmann PartmbB

(57) **Abstract**

The present disclosure relates to supports comprising an optically transparent substrate and at least one sample spot on the surface of the support, devices for determining a single molecule event comprising said supports and methods for determining a single molecule event wherein said supports are used. The substrate is at least partially coated with a layer of diamond-like and/or amorphous carbon and the layer of diamond-like and/or amorphous carbon does not extend over the at least one sample spot.

## Description

The present disclosure relates to supports comprising an optically transparent substrate and at least one sample spot on the surface of the support, devices for analyzing single molecule events comprising said supports and methods for analyzing single molecule events wherein said supports are used.

### Background

Critical to the development of biomedical devices and systems, from biosensors to drug delivery, is the use of protein-resistant surfaces. This is also an absolute requirement for single molecule studies of biomolecular interactions, where any signal from non-specifically bound proteins on the surface of the test device interferes with the measurement.

One of the most commonly used passivation techniques is the use of protein-repellent coatings wherein polyethylene glycol (PEG) molecules are chemically linked to the underlying substrate and arranged in a thin brush-like layer in many configurations [1]. PEG passivation, however, is often defective, and many techniques have been developed to improve its coverage and effectiveness [2-4]. Other known organic passivating films have similar deficiencies [4].

Inorganic film of diamond-like carbon (DLC) or amorphous carbon are highly resistant to protein adsorption. The carbon films are highly uniform and are extremely robust, with mechanical strength far exceeding that of organic layers. They are relatively immune from environmental or ambient conditions (e.g., they do not have to be stored in water), properties that also make them appropriate for reuse and have been found to resist adhesion of proteins, and they are considered excellent candidates as coatings for biomedical devices such as implants, etc. [5, 6]. DLC films possess outstanding mechanical and tribological properties [7, 8], and have a low surface energy. They have found use as an anti-wear coating in a wide variety of applications [9].

Further reduction in surface energy, and thus in wettability, may be achieved by introducing fluorine into the film. The fluorine may be introduced either during DLC deposition by CVD [10] or by exposure to a fluorine-containing plasma from fluorocarbon-based gases or the like (e.g., C₄F₈, CHF₃, NF₃, ₒᵣ SF₆). Fluorine atoms are present at [10] or within [11, 12] an atomic distance of the surface. The result is a highly hydrophobic (or superhydrophobic) surface [11, 12] that is resistant to adhesion of many species and, in particular, has been demonstrated to be extremely resistant to the adhesion of cells and biomolecules [6, 13, 14].

The present inventors are, however, not aware of using optically transparent surfaces coated with inorganic carbon layers in in single molecule studies of biomolecular interactions involving an optical detection in a reaction space located on a coated support.

### Summary of the Invention

### Support coated with a carbon film

In a first aspect, the present disclosure relates to a support that is area-selective coated with a carbon film and devices and methods for analyzing events associated with emission of an electromagnetic radiation, e.g., single molecule events wherein said support is used.

According to this aspect, the support comprises a substrate having a surface coated with a film of diamond-like carbon (DLC) and/or amorphous carbon in an area-selective manner, rendering it resistant to adhesion of proteins and other biological moieties in the coated regions, while allowing selective binding of said biological moieties to non-coated regions. The carbon film may be deposited by any suitable method including, but not limited to, physical vapor deposition, chemical vapor deposition and/or atomic layer deposition on the surface of the substrate. Its anti-adhesion properties may be further enhanced by fluorination by, e.g., treatment with a fluorine-based plasma. The carbon film is mechanically robust and chemically stable, and it can withstand multiple sequences of use with minimal cleaning and/or reactivation steps. Thus, carbon-coated surfaces can replace surfaces treated with conventional protein-repellent reagents, such as poly-ethylene glycol (PEG).

In a first embodiment of this aspect, the present disclosure relates to a support comprising a substrate and at least one sample spot on the surface of the support, wherein the substrate is at least partially coated with a layer of diamond-like and/or amorphous carbon and wherein the layer of diamond-like and/or amorphous carbon does not extend over the at least one sample spot. In a particular embodiment, the substrate is an optically transparent substrate.

A further embodiment relates to the use of the above support for analyzing an event at the at least one sample spot wherein said event is associated with emission of an electromagnetic radiation. In a particular embodiment, the event is a single molecule event.

In a further embodiment, the support is reusable after being subjected to a cleaning procedure wherein a biological moiety attached to the at least one sample spot is removed.

A further embodiment relates to a method of manufacturing the above support comprising:
(i) providing a substrate comprising at least one sample spot on the surface of said substrate, and
(ii) coating the surface of the substrate with a layer of diamond-like and/or amorphous carbon,
wherein the material of the sample spot is selected such that is not susceptible to the coating in step (iii), whereby the layer of diamond-like and/or amorphous carbon does not extend over the at least one sample spot.

A further embodiment relates to a method of manufacturing the above support comprising:
(i) coating the surface of a substrate with a layer of diamond-like and/or amorphous carbon, and
(ii) depositing at least one sample spot on the surface of said layer.

A further embodiment relates to a method of manufacturing the above support comprising:
(i) coating the surface of a substrate with a layer of diamond-like and/or amorphous carbon,
(ii) removing said layer in a selected area of the surface, and
(iii) depositing at least one sample spot in said selected area where said layer has been removed.

A further embodiment relates to a method for analyzing an event comprising:
(i) providing the above support,
(ii) immobilizing a biological moiety, particularly a biomolecule on the at least one sample spot of the support, and
(i) analyzing an event associated with said biological moiety, particularly an event associated with said biomolecule, by detecting electromagnetic radiation from said sample spot.

In particular embodiments, the event is a single molecule event, and the biomolecule is a single biomolecule.

A further embodiment relates to a device for analyzing an event comprising:
(i) the above support adapted for immobilizing a biological moiety, particularly a biomolecule on the at least one sample spot of the support,
(ii) means for illuminating the at least one sample spot of the support with radiation, and
(iii) means for analyzing an event on said the at least one sample spot by detecting an electromagnetic radiation from said spot.

In particular embodiments, the event is a single molecule event, and the biomolecule is a single biomolecule.

### Cleaning of a previously used support

A second aspect described herein is a method of cleaning a previously used support suitable for devices and methods for analyzing an event, e.g., a single molecule event, wherein said event is associated with emission of an electromagnetic radiation.

If not indicated differently, the features of the first aspect also apply to the second aspect.

An embodiment of the second aspect relates to a method of cleaning a previously used support comprising:
(i) providing a support comprising a substrate and at least one sample spot on the surface of the support, wherein a biological moiety is attached to the at least one sample spot.
(ii) subjecting the support from step (i) to a treatment with an alkaline solution, wherein the treatment is performed at an elevated temperature;
(iii) optionally rinsing the support after step (ii);
(iv) subjecting the support after step (ii) or (iii) to a treatment with an acidic/oxidative solution comprising a strong inorganic acid and a peroxide;
(v) optionally rinsing the support after step (iv);
(vi) optionally drying the support after step (iv) or (v) with an inert gas;
(vii) subjecting the support after step (iv), (v) or (vi) to a plasma treatment.

In particular embodiments, the substrate is an optically transparent substrate.

In further embodiments of this aspect, a biological moiety such as a biomolecule is attached to the cleaned support after step (vii). Further, the support surface surrounding the sample spots may be passivated, i.e., treated with a protein adhesion repellent, in order to inhibit adhesion of biological moieties such as biomolecules and/or other sample constituents. Thereafter, the support is ready for use in a new analysis of an event, e.g., a single molecule event.

### Items of the Specification

In the following, particular items of the specification are described herein:
1. A support comprising a substrate and at least one sample spot on the surface of the support,
   wherein the substrate is at least partially coated with a layer of diamond-like and/or amorphous carbon and wherein the layer of diamond-like and/or amorphous carbon does not extend over the at least one binding spot.
2. The support of item 1, which is at least substantially planar.
3. The support of item 1 or 2, which is structured.
4. The support of any one of the preceding items, wherein the substrate is optically transparent.
5. The support of any one of the preceding items, wherein the substrate comprises a material having a refractive index of at least 1.01.
6. The support of any one of the preceding items, wherein the substrate comprises a non-electrically conductive material.
7. The support of any one of the preceding items, wherein the substrate comprises a material selected from the group consisting of silica, quartz, and glass.
8. The support of any one of the preceding items, wherein the substrate has a thickness of about 10 µm to about 5 mm, particularly about 20 µm to about 2 mm.
9. The support of any one of the preceding items, which comprises a plurality of sample spots, e.g., at least 10, at least 100, at least 1,000 or at least 10,000 sample spots.
10. The support of any one of the preceding items, wherein the at least one sample spot comprises at least one electrically conductive material, e.g., a metal including a pure metal or a combination of metals, e.g., an alloy or mixture of a plurality of different metals.
11. The support of item 10, wherein the at least one metal is capable of forming a bond with sulfur, e.g., in the form of a thiol or a disulfide.
12. The support of any one of items 10-11, wherein the at least metal has a positive electrochemical potential.
13. The support of any one of items 10-12, wherein the at least one metal is selected from Au, Cu, Ni, Pt, Pd, Rh, Ir, Os, Ru and any combination comprising at least two of said metals.
14. The support of any one of items 1-9, wherein the at least one sample spot comprises at least one metal oxide such as TiO₂, NiO, or ITO.
15. The support of any one of the preceding items, wherein the at least one sample spot has a diameter of about 1 nm to about 100 µm, particularly a diameter of about 2 nm to about 50 µm.
16. The support of any one of the preceding items, wherein the at least one sample spot has a diameter of about 1 nm to about 100 nm, particularly a diameter of about 2 nm to about 50 nm.
17. The support of any one of the preceding items, wherein the at least one sample spot has an upper face distal to the substrate and wherein the upper face of is of even elevation with the surrounding layer of diamond-like and/or amorphous carbon.
18. The support of any one of items 1-17, wherein the at least one sample spot has an upper face distal to the substrate and wherein the upper face of the sample spot is above the surrounding layer of diamond-like and/or amorphous carbon.
19. The support of any one of items 1-17, wherein the at least one sample spot has an upper face distal to the substrate and wherein the upper face of the sample spot is below the surrounding layer of diamond-like and/or amorphous carbon.
20. The support of any one of the preceding items, wherein the at least one sample spot has a lower face proximal to the substrate and an upper face distal to the substrate, wherein the distance between the lower and upper face defines the height of the sample spot, and wherein the height is about 50 pm to about 500 nm, particularly about 100 pm to about 20 nm, and more particularly about 500 pm to about 10 nm, e.g., about 2 nm.
21. The support of any one of the preceding items, wherein the at least one sample spot extends through the carbon layer such that its lower face is in direct contact with the surface of the substrate.
22. The support of any one of items 1-20, wherein the at least one sample spot does not extend through the carbon layer such that its lower face is in direct contact with the surface of the carbon layer.
23. The support oof any one of items 1-20, wherein the at least one sample spot is located upon a pillar that has been etched into a planar substrate, wherein the layer of diamond-like and/or amorphous carbon coats the sidewalls of the pillar as well as the planar surface of the substrate.
24. The support of any one of the preceding items, wherein the layer of diamond-like and/or amorphous carbon has a thickness of about 0.3 nm to about 200 µm, particularly about 5 nm to about 100 µm, and more particularly about 1 µm to about 50 µm.
25. The support of any one of the preceding items, wherein the layer of diamond-like and/or amorphous carbon is a fluorinated diamond-like and/or fluorinated amorphous carbon.
26. The support of any one of the preceding items, wherein the at least one sample spot is adapted for the attachment, e.g., adapted for the covalent or con-covalent attachment of a biological moiety selected from a biomolecule, a cell fraction, a cell organelle, or a cell.
27. The support of any one of the preceding items, wherein the at least one sample spot is adapted for the attachment, e.g., adapted for the covalent or con-covalent attachment of a single biomolecule.
28. The support of any one of the preceding items, wherein a biological moiety selected from a biomolecule, a cell fraction, a cell organelle, or a cell is attached to at least one of the sample spots.
29. The support of any one of the preceding items, wherein a single biomolecule is attached to at least one of the sample spots.
30. The support of any one of items 26-29, wherein the biomolecule is selected from polypeptides, nucleic acids, carbohydrates, and any combination thereof.
31. The support of any one of items 26-30, wherein the biomolecule is a nucleic acid-polymerizing enzyme, e.g., an RNA polymerase or a DNA-polymerase, or a nucleic acid-degrading enzyme, e.g., an exonuclease.
32. Use of a support of any one of items 1-31 for analyzing an event occurring on the at least one sample spot, wherein said event is associated with emission of an electromagnetic radiation.
33. Use of a support of any one of items 1-31 for analyzing a single molecule event occurring on the at least one sample spot, wherein said single molecule event is associated with emission of an electromagnetic radiation.
34. Use of a support of any one of items 1-31 for separately analyzing a plurality of events, particularly single molecule events each occurring on at least one sample spot, wherein said single molecule events are associated with emission of an electromagnetic radiation.
35. The use of item 34 wherein a plurality of events, particularly single molecule events is analyzed in parallel.
36. The use of any one of items 32-35 wherein the single molecule event comprises a nucleic acid sequence determination.
37. The use of item 36, wherein the nucleic acid sequence determination comprises
   - providing at a sample spot (i) a single nucleic acid molecule, (ii) a nucleic acid-synthesizing enzyme molecule and/or a nucleic acid degrading enzyme molecule, and (iii) fluorescence labelled nucleotide building blocks in free form and/or incorporated into the nucleic acid molecule,
   - conducting an enzymatic reaction, wherein nucleotide building blocks are incorporated into and/or cleaved off from said single nucleic acid molecule, and
   - individually determining the base sequence of the nucleic acid molecule on the basis of the time-dependent fluorescence change, caused when nucleotide building blocks are incorporated into and/or cleaved off from said single nucleic acid molecule.
38. The use of item 37, wherein a nucleic acid-synthesizing enzyme molecule and/or a nucleic acid degrading enzyme molecule is immobilized on said sample spot.
39. The use of any one of items 32-38, wherein the support has been previously employed for the analysis of an event, e.g., a single molecule event and thereafter been subjected to a cleaning procedure wherein a biological moiety such as a biomolecule attached to the at least one sample spot has been removed.
40. A method of manufacturing a support of any one of items 1-31 comprising:
   (i) providing a substrate comprising at least one sample spot on the surface of said substrate, and
   (ii) coating the surface of the substrate with a layer of diamond-like and/or amorphous carbon,
   wherein the material of the sample spot is selected such that is not susceptible to the coating in step (iii), whereby the layer of diamond-like and/or amorphous carbon does not extend over the at least one sample spot.
41. A method of manufacturing a support of any one of items 1-31 comprising:
   (i) coating the surface of a substrate with a layer of diamond-like and/or amorphous carbon, and
   (ii) depositing at least one sample spot on the surface of said layer.
42. A method of manufacturing a support of any one of items 1-31 comprising:
   (i) coating the surface of a substrate with a layer of diamond-like and/or amorphous carbon,
   (ii) removing said layer in a selected area of the surface, and
   (iii) depositing at least one sample spot in said selected area where said layer has been removed.
43. A method for analyzing an event, e.g., a single molecule event comprising:
   (ii) providing a support of any one of items 1-31,
   (iii) immobilizing a biological moiety, particularly a single biomolecule on the at least one sample spot of the support, and
   (iv) analyzing an event, associated with said biological moiety, particularly a single molecule event associated with said single biomolecule, by detecting electromagnetic radiation from said sample spot.
44. The method of item 43 wherein the single molecule event comprises a sequence analysis of a single nucleic acid molecule.
45. A device for analyzing a single molecule event comprising:
   (i) a support of any one of items 1-31,
   (ii) means for illuminating the at least one sample spot on the support with radiation, and
   (iii) means for analyzing a single molecule event on said the at least one sample spot by detecting electromagnetic radiation from said sample spot.
46. The device of item 45 adapted for the sequence analysis of a single nucleic acid molecule.
47. A method of cleaning a previously used support comprising:
   (i) providing a substrate and at least one sample spot on the surface of the support, wherein a biological moiety is attached to the at least one sample spot.
   (ii) subjecting the support from step (i) to a treatment with an alkaline solution comprising an alkaline phosphate, e.g., potassium phosphate and optionally a surfactant and/or a chelator, wherein the treatment is performed at an elevated temperature, particularly under boiling;
   (iii) optionally rinsing the support after step (ii) e.g., with water;
   (iv) subjecting the support after step (ii) or (iii) to a treatment with an acidic/oxidative solution comprising a strong inorganic acid, e.g., sulfuric acid and a peroxide, e.g., hydrogen peroxide;
   (v) optionally rinsing the support after step (iv) e.g., with water and/or an anhydrous organic solvent, e.g., ethanol;
   (vi)optionally drying the support after step (iv) or (v) with an inert gas, e.g., Ar, N₂ or any mixture thereof;
   (vii) subjecting the support after step (iv), (v) or (vi) to a plasma treatment, particularly to an O₂ plasma treatment.
48. The method of item 47, wherein the support has been previously used for analyzing an event, e.g., a single molecule event, wherein said event is associated with emission of an electromagnetic radiation.
49. The method of item 47 or 48, wherein the support in step (i) comprises a substrate and at least one sample spot on the surface of the support, wherein the substrate is at least partially coated with a layer of diamond-like and/or amorphous carbon and wherein the layer of diamond-like and/or amorphous carbon does not extend over the at least one sample spot.
50. The method of item 47 or 48, wherein the support comprises a substrate and at least one sample spot on the surface of the support, wherein the substrate is at least partially coated with a layer of an organic passivating material, and wherein the layer of organic passivating material does not extend over the at least one sample spot.
51. The method of any one of items 47-50, comprising attaching a biological moiety such as a biomolecule to the cleaned support after step (vii).
52. The method of item 51 comprising optionally passivating the substrate and attaching a biomolecule to the at least one sample spot, wherein the support is ready for use in a new analysis of an event, e.g., a single molecule event.
53. The method of item 51 or 52 comprising
   (viii) activating the surface of the at least one sample spot, e.g., subjecting the support after step (vii) to a treatment with a thiol reagent;
   (ix) optionally rinsing the support after step (viii) e.g., with an organic solvent, such as anhydrous ethanol;
   (x) optionally drying the support after step (viii) or (ix) with an inert gas, e.g., Ar, N₂ or any mixture thereof;
   (xi)optionally regenerating the surface of said support, e.g., passivating the substrate, e.g., by treatment with a PEGylating reagent, such as an alkoxy PEG silane, e.g., a methoxy PEG silane, or by coating the surface of said support with a layer of diamond-like and/or amorphous carbon;
   (xii) optionally rinsing the support after step (xi) e.g., with an organic solvent, e.g., acetone and/or anhydrous ethanol;
   (xiii) optionally drying the support after step (xi) or (xii) with an inert gas, e.g., Ar, N₂ or any mixture thereof; and
   (xiv) binding a biomolecule to the activated surface of the at least one sample spot, wherein the support is ready for use in a new determination of a single molecular event.

### Detailed Description

The present disclosure relates to a support comprising a substrate coated with a layer of diamond-like carbon and/or amorphous carbon (in the following also designated "carbon film". In certain embodiments, the support is a substantially planar support, i.e., it does not comprise elevations or recesses of about 1000 nm or more or of about 100 nm or more. In further embodiments, the support is a structured support, e.g., a support comprising recesses such as wells that may have a volume of about 5 × 10⁻²⁴ liters to 1 × 10⁻¹⁵ liter, or pillars of height of about 1 nm to 500 nm. In principle, the support may have any design, as long as a reaction space can be formed which enables the occurrence of a single molecule event on a sample spot present on the support.

In certain embodiments, the substrate is an optically transparent material, i.e., a material that is substantially transparent for electromagnetic radiation, e.g., radiation in the visible range and/or radiation in the near-infrared range. In certain embodiments, the substrate comprises a material having an absolute refractive index of at least 1.01, e.g., about 1.5 to about 3 in the visible range or about 1.5 to about 4 in the near-infrared range. In further embodiments, the substrate is an optically opaque material, e.g., a metal or semi-metal such as silicon.

In particular embodiments, the substrate comprises a non-electrically conductive material. Specific examples are glass, quartz, plastic, metal oxide-based materials, e.g., silicon dioxide-based materials such as glass, silica, or quartz, or composites comprising said materials. In further embodiments, the substrate comprises an electrically conductive material, e.g., an optically transparent material such as indium tin oxide.

Typically, the substrate has a thickness of about 10 µm to about 5 mm, particularly about 20 µm to about 2 mm.

According to the present disclosure, the substrate is coated with a layer of a carbon film. In certain embodiments, the carbon film forms a continuous layer on the surface except for the area of the at least one sample spot.

The carbon film may be deposited on the substrate by physical vapor deposition, chemical vapor deposition (CVD) or atomic layer deposition (ALD) techniques. In CVD, a surface is exposed to a volatile gas that includes a precursor of the desired deposition material. The precursor is chemically decomposed (typically at elevated temperature, although there are many variations of CVD in which additional factors, such as electromagnetic fields, play a role), and the desired material is deposited on the surface, whilst the remaining, volatile components are removed by a vacuum pump. CVD films ranging from a few nanometers to microns in thickness are achievable. In ALD, films are deposited one atomic layer at a time. The deposition process involves first exposing the surface to a linker molecule that forms a bond to the surface. A "priming material" (e.g., water) is introduced to render the linker available for reaction with the desired deposition material. The desired deposition material is then introduced, typically from the same sort of precursor as is used in CVD. This binds with the linker molecule such that a single atomic layer is formed in a self-limiting fashion. A film is thus built up by a repetitive sequence of the above. Because of the chemical interactions involved in this process, ALD is more limited that CVD in terms of the materials that can be deposited in this way, and it can be deposited in a materials-selective manner. It is also best suited to the formation of films with thicknesses ranging from a few nanometers to a few tens of nanometers.

In certain embodiments, the carbon film is fluorinated. Fluorine atoms may be introduced by known procedures, e.g., during DLC deposition by CVD or by exposure to a fluorine-containing plasma from fluorocarbon-based gases or the like (e.g., C₄F₈, CHFs, NFs, or SF₆).

In certain embodiments, the carbon film including a fluorinated carbon film has a thickness of about 0.3 nm to about 200 µm, particularly about 10 nm to about 100 µm, and more particularly about 1 µm to about 50 µm.

Described herein are surfaces that are area-selective coated such that they contain the surface of the carbon a film as protein-resistant region and at least one sample spot adapted for attaching a biological moiety, e.g., a biomolecule. In certain embodiments, the support comprises a plurality of sample spots, e.g., at least 10, at least 100, at least 1,000, at least 10,000 sample spots, or at least 100,000 spots. In certain embodiments, the support may comprise up 10⁶ or 10⁹ or even more sample spots.

In certain embodiments, a sample spot comprises or consists of at least one electrically conductive material, e.g., a metal including a single metal or a combination of metals, e.g., an alloy or mixture of a plurality of different metals. For example, a metal that is capable of attachment to a sulfur containing moiety, e.g., in the form of a thiol or a disulfide, or a metal that is capable of attachment to a chelating moiety, e.g., a poly-histidine tag, is suitable. In certain embodiments, the metal has a positive electrochemical potential. Specific examples of suitable metals include but are not limited to Au, Cu, Ni, Pt, Pd, Rh, Ir, Os, Ru, and any combination thereof comprising at least two of said metals.

In certain embodiments, a sample spot comprises or consists of at least one at least one metal oxide including a single metal oxide or a combination of metal oxides. For example, a metal oxide that is capable of attachment to a phosphorus containing moiety, e.g., in the form of a phosphonic acid or phosphonic acid ester, or a metal oxide that is capable of attachment to a chelating moiety, e.g., a poly-histidine tag, is suitable. Specific examples of suitable metal oxides include TiO₂ and NiO.

In further embodiments, a sample spot comprises or consists of at least one non-electrically conductive material.

Sample spots may be prepared by vapour deposition of metals, which are vaporized on the support covered by a grid mask, which may be produced by electron beam lithography or equivalent technologies. The size of holes in the grid mask may correspond to the size of the spots on the support surface. Alternatively, the spots on the support may be prepared by site-specific deposition of nanoparticles, e.g., having a size of 2-10 nm, by precision pipetting of particles on the support, particularly on a support having a planar surface.

The sample spot may have a size that is suitable for the attachment of biological moieties such as biomolecules, cell fractions, cell organelles or cells, e.g., prokaryotic, or eukaryotic cells. In certain embodiments, a sample spot has a diameter of about 1 nm to about 100 µm, particularly a diameter of about 2 nm to about 50 µm.

In particular embodiments, a sample spot has a size that is suitable for the attachment of a single biomolecule. In those embodiments, a sample spot has a diameter of about 1 nm to about 100 nm, particularly a diameter of about 2 nm to about 50 nm.

In certain embodiments, the sample spot is a separate object on the surface of the substrate. In certain embodiments, the sample spot has a lower face proximal to the substrate and an upper face distal to the substrate, wherein the distance between the lower and upper face defines the height of the sample spot. In particular embodiments, the height is about 50 pm to about 500 nanometers, particularly about 100 pm to about 20 nm, and more particularly about 500 pm to about 10 nm, e.g., about 2 nm.

There are several options for the arrangement of the sample spot, the carbon layer and the substrate.

In certain embodiments, the upper face of the sample spot is of even elevation with the surrounding carbon film. In certain embodiments, the upper face of the sample spot is above the surrounding carbon film. In certain embodiments, the upper face of the sample spot is below the surrounding carbon film. The distance between the upper face of the sample spot and the carbon film may be in the range of 0.1 nm to 500 nm.

In certain embodiments, the sample spot extends through the carbon layer such that its lower face is in direct contact with the surface of the substrate. In certain embodiments, the sample spot does not extend through the carbon layer such that its lower face is in direct contact with the surface of the carbon layer. In certain embodiments, the sample spot is located upon a pillar that has been etched into a planar substrate, wherein the layer of diamond-like and/or amorphous carbon coats the sidewalls of the pillar as well as the planar surface of the substrate.

The sample spot is adapted for the attachment of a biological moiety, e.g., a biomolecule, a cell fraction, a cell organelle, or a cell, e.g., a prokaryotic, or eukaryotic cell. In certain embodiments, the sample spot is adapted for the attachment of a single biomolecule, e.g., by a covalent or non-covalent attachment. The biomolecule may be selected from polypeptides, nucleic acids, carbohydrates, and any combination thereof. In certain embodiments, wherein the biomolecule is a nucleic acid-polymerizing enzyme, e.g., an RNA polymerase or a DNA-polymerase, or a nucleic acid-degrading enzyme, e.g., an exonuclease.

In certain embodiments, a biological moiety such as a biomolecule is directly attached to the sample spot, e.g., by providing a biological moiety such as a biomolecule having an anchor group suitable for covalent or non-covalent attachment to the surface of the sample spot, particularly a sulfur-containing group such as a thiol group -SH, a substituted thiol group -SR, wherein R is an organic residue, e.g., a C₁-C₄ alkyl group or a disulfide group -S-S-, or a phosphorus-containing group. Yet another alternative is immobilization in which it is possible to mediate the binding of a biological moiety such as a biomolecule via reactive silane groups on a silica surface.

In certain embodiments, a biological moiety such as a biomolecule is indirectly attached to the sample spot, e.g., by non-covalent high affinity linkage to a coating on the surface of the sample spot. In those embodiments, a biological moiety such as a biomolecule may be provided that has a reactive tag, e.g., a biotin, hapten, poly(histidine) tag or carbohydrate group, capable of forming a high-affinity linkage with a complementary reactive moiety attached to the surface of the sample spot, e.g., streptavidin, antibody, lectin, etc.

In certain embodiments, immobilizing the biological moiety to the support may involve a coupling reaction between 2 bio-orthogonal reactive groups, i.e., groups not occurring in biomolecules to be attached to the sample spot. In certain embodiments, the coupling reaction is a Click reaction, e.g., a reaction between an azide group and an alkyne group, e.g., a terminal alkyne group or a strained alkyne group, e.g., a cyclooctyne group. In certain embodiments, the reaction product of the coupling reaction comprises a triazole group.

In certain embodiments, a biological moiety such as a biomolecule may be provided that has a bio-orthogonal group, e.g., an azide group or an alkyne group, capable of forming a covalent linkage with a complementary bio-orthogonal group attached to the surface of the sample spot.

The support as disclosed herein may be manufactured by different procedures as outlined in the following.

In certain embodiments, manufacture of the support comprises:
(i) providing a substrate comprising at least one sample spot on the surface of said substrate, and
(ii) coating the surface of the substrate with a layer of the carbon film, e.g., by physical or chemical vapor deposition (CVD) or by atomic layer deposition (AVD),
wherein the material of the sample spot is selected such that is not susceptible to the coating in step (iii), whereby the layer of diamond-like and/or amorphous carbon does not extend over the at least one sample spot.

According to this embodiment, examples of suitable materials of the sample spot include, but are not limited to Au, Cu, and Ni as described in [7] and [11].

In certain embodiments, manufacture of the support comprises:
(i) coating the surface of a substrate with a carbon film, e.g., by physical or chemical vapor deposition (CVD) or by atomic layer deposition (AVD), and
(ii) depositing at least one sample spot on the surface of said layer.

The support of the present disclosure is suitable for analyzing an event occurring on a sample spot, wherein the event is associated with the emission of an electromagnetic radiation from the sample spot. In particular embodiments, the event encompasses a reaction of a biological moiety which is associated with emission of a characteristic electromagnetic radiation. In particular embodiments, the event is a single molecule event.

In particular embodiments, the support of the present disclosure is suitable for analyzing an event, e.g., a single molecule event occurring on the at least one sample spot, particularly for separately analyzing a plurality of single molecule events each occurring on at least one sample spot and even more particularly for separately analyzing a plurality of single molecule events is analyzed in parallel. In particular embodiments, the single molecule event comprises a nucleic acid sequence determination

A further embodiment relates to a method for analyzing an event comprising:
(iii) providing the above support,
(iv) immobilizing a biological moiety, particularly a biomolecule on the at least one sample spot of the support, and
(v) analyzing an event associated with said biological moiety, particularly an event associated with said single biomolecule by detecting electromagnetic radiation from said sample spot.

In particular embodiments, the event is a single molecule event, and the biomolecule is a single biomolecule.

In particular embodiments, the single molecule event comprises the sequence analysis of a single nucleic acid molecule.

A further embodiment relates to a device for analyzing an event comprising:
(iv) the above support adapted for immobilizing a biological moiety, particularly a biomolecule on the at least one sample spot of the support,
(v) means for illuminating the at least one sample spot of the support with radiation, and
(vi) means for analyzing an event on said the at least one sample spot by detecting electromagnetic radiation from said spot.

In particular embodiments, the event is a single molecule event, and the biomolecule is a single biomolecule.

In particular embodiments, the device is adapted for the sequence analysis of a single nucleic acid molecule.

Methods and devices for analyzing single molecule events are e.g., disclosed in WO 2002/097406, WO 2003/052137, WO 2006/013110, WO 2013/131888, WO 2015/104245, WO 2017/001407, and WO 2018/104301, the contents of which are herein incorporated by reference.

For the analysis of a single molecule event, the biomolecules are located at the sample spots on the support. There they are in contact with a sample liquid, which contains the free reaction partners. Thereby, one or more reaction spaces are defined. Particularly at least 100, at least 1000, or at least 10 000, and up to more than 10⁶ molecules may be analysed on a single support, e.g., a single planar support

A nucleic acid molecule whose sequence is to be determined may be selected, for example, from DNA molecules such as genomic DNA fragments, cDNA molecules, plasmids, etc., or else from RNA molecules such as mRNA molecules. The nucleic acid molecule may originate from genomic or expression libraries, generated from cells or organisms, e.g., eukaryotic or prokaryotic cells or organisms. This allows parallel sequencing of a plurality of different nucleic acid template molecules, e.g., at least 10, 100, 1.000 or 10.000 and up to 100.000, 10⁶ or 10⁷ or even more different nucleic acid molecules.

The nucleic acid molecules to be sequenced may be single-stranded nucleic acid molecules in a linear or circular form, e.g., in a covalently linked circular form. In order to obtain a circular nucleic acid template, a linear nucleic acid molecule may be subjected to a circularization procedure and optionally a strand-separation procedure during sample preparation. Circularization may be effected by ligation according to known protocols, e.g., using DNA or RNA ligases. In some embodiments, an adaptor and/or identifier molecule, i.e., a nucleic acid molecule of known sequence, may be coupled to the nucleic acid molecule.

The sequence determination may comprise nucleic acid elongation and/or nucleic acid degradation. The sequencing process includes one or more sequencing cycles.

The nucleic acid-synthesizing enzyme molecules are capable of elongating a primer annealed to a nucleic acid template molecule. Primer elongation may be carried out by progressively incorporating individual nucleotide building blocks at the 3'-terminus of a growing nucleic acid chain, wherein a nucleic acid molecule complementary to the sequence of the circular nucleic acid template is generated. The nucleic acid-synthesizing enzymes are selected from polymerases capable of a template specific nucleic acid polymerization, preferably from DNA polymerases and RNA polymerases, e.g., natural or modified polymerases, including thermostable DNA polymerases.

Specific examples of suitable DNA polymerases include Taq polymerases, exonuclease-deficient Taq polymerases, E.coli DNA polymerase I, Klenow fragment, reverse transcriptase, Φ29-related polymerases including wild-type Φ29 polymerase and derivatives of such polymerases, such as exonuclease-deficient forms, T7 DNA polymerase, T5 DNA polymerase, an RB69 polymerase and others.

Nucleic acid-degrading enzyme molecules are capable of progressively cleaving off individual nucleotide building blocks from a nucleic acid molecule. Preferably exonucleases, more preferably single-strand exonucleases which degrade in the 3'→5' direction or in the 5'→3' direction are used. Exonucleases which are particularly preferably used are 3'→5' exonucleases such as E.coli exonuclease I and E.coli exonuclease III, and 5'→3' exonucleases such as T7 exonuclease, E.coli exonuclease II and E.coli exonuclease VIII. Further, the exonuclease activities of various polymerases, e.g., the Klenow fragment, Taq polymerase or T4 polymerase may be used.

The nucleic acid-synthesizing enzyme molecules are contacted with a linear or circular nucleic acid template molecule, e.g., a single-stranded DNA or RNA molecule, and a primer molecule annealed to the nucleic acid template molecule or capable of annealing thereto. The primer molecule is preferably a single-stranded nucleic acid or nucleic acid analogue molecule having a free 3'-end which can be extended by an enzymatic reaction catalyzed by the immobilized nucleic acid-synthesizing enzyme molecules. The length of the primer molecule is selected to allow effective annealing to the template under reaction conditions. Usually, the length of the primer molecule is at least 8, at least 10, at least 12 or at least 15 nucleotides and e.g., up to 20, 25, 50 or 100 nucleotides, or even higher. In some embodiments, the primer is resistant against digestion by nucleic acid-degrading enzyme molecules, e.g., by incorporating nucleotide analogue building blocks and/or linkages between nucleotide building blocks, which are stable against degradation. In other embodiments, the primer is sensitive against digestion by nucleic acid-degrading enzyme molecules.

The sequence of the primer is selected in that it effectively anneals under reaction conditions to the template molecule. For instance, the primer may be a universal degenerated primer capable of statistically annealing to unknown nucleic acid sequences. In other embodiments, the primer may be capable of annealing to a known sequence portion of the nucleic acid template molecule. In this embodiment, a known adaptor and/or identifier sequence may be incorporated into the nucleic acid template molecule. The primer may be unlabelled or comprise fluorescent labelling groups.

Further, the presence of nucleotide building blocks carrying at least one fluorescent labelling group is required. Preferably, each different nucleotide building block (A, G, C, T/U) contains a different fluorescent labelling group.

The fluorescent labelling groups may be selected from known fluorescent labelling groups used for labelling biopolymers, particularly nucleic acids, such as, for example, fluorescein dyes, rhodamines, oxazines, for example Evoblue or Gnothis Blue, phycoerythrin, Cy3, Cy5, IR dyes or derivatives thereof, etc.

The nucleotide building blocks may carry (i) a fluorescence labelling group which remains with the building block when the building block is incorporated into a nucleic acid molecule during a primer elongation catalyzed by a nucleic acid-synthesizing enzyme molecule, and/or (ii) a fluorescence labelling group which is cleaved off from the building block when the building block is incorporated into a nucleic acid molecule during a primer elongation catalyzed by a nucleic acid-synthesizing enzyme molecule. Fluorescence labelling groups remaining with the building block are preferably attached to the α-phosphate group, to the sugar and/or to the nucleobase group.

In particular embodiments, fluorescence labelling groups remaining with the building block are attached to the nucleobase, e.g., via a linker which may have a chain-length of up to 15, preferably of 10-12 carbon atoms, optionally including heteroatoms, e.g. N, O or S atoms. Fluorescence labelling groups which are cleaved off when the building block is incorporated into a nucleic acid molecule may be attached to a terminal phosphate group, e.g., of a polyphosphate building block including, but not limited to a hexa-, penta-, tetra- or triphosphate building block such as the γ-phosphate group of a triphosphate building block. In certain embodiments, building blocks are selected which contain both (i) a fluorescence labelling group remaining after incorporation and (ii) a fluorescence labelling group cleaved off during incorporation. In this case, fluorescence groups capable of interacting with each other, e.g., by quenching and/or energy transfer, may be selected.

The nucleic acid molecules to be sequenced will contain fluorescent labelling groups in case the nucleic acid molecule is subjected to direct sequencing using a nucleic acid-degrading enzyme molecule. On the other hand, the nucleic acid molecule to be sequenced my not contain fluorescent labelling groups, if the nucleic acid molecule is used as a template in a primer elongation.

The sequencing procedure may involve a step of generating nucleic acid molecules having incorporated nucleotide building blocks in a primer elongation catalyzed by the nucleic acid-synthesizing enzyme molecules and/or a second step of cleaving off individual nucleotide building blocks from the generated nucleic acid molecules catalyzed by nucleic acid-degrading enzyme molecules. Dependent on the type of fluorescence labels, nucleic acid sequence determination may be carried out during primer elongation and/or during degradation.

Sequence determination during the primer elongation involves the use of nucleotide building blocks carrying a fluorescence-labelling group which is cleaved off from the building block when it is incorporated into a nucleic acid molecule. In this case, a time-dependent fluorescence change caused by cleaving off the fluorescence-labelling group from the nucleotide building block may be determined. Sequence determination during nucleic acid degradation involves the use of a nucleotide building block, which carries a fluorescence-labelling group which remains with the building block when it is incorporated into a nucleic acid molecule. Progressive cleavage of individual nucleotide building blocks from the nucleic acid molecules causes a time-dependent change of fluorescence when the labelled nucleotide building block is liberated from the nucleic acid molecule. In certain embodiments, it is also possible to carry out a sequence determination during elongation and degradation, i.e., when using nucleotide building blocks, which both carry a fluorescence-labelling group remaining with the building block and a fluorescence-labelling group which is cleaved off from the building block when the building block is incorporated into a nucleic acid molecule. In this embodiment, both fluorescent groups may be the same or different.

In some embodiments, the method involves one or more cycles of nucleic acid-synthesis and nucleic acid-degradation in order to determine the base sequence of a nucleic acid molecule template. The nucleic acid synthesis involves an elongation of the primer annealed to the nucleic acid template molecule catalyzed by the nucleic acid-synthesizing enzyme molecule, wherein a nucleic acid molecule complementary to the sequence of the nucleic acid template is generated. In the next step, the generated nucleic acid molecule is degraded by a nucleic acid-degrading enzyme molecule.

When a nucleotide building block is incorporated into an elongated nucleic acid molecule, a time dependent change in the fluorescence may occur, which can be detected as indicated above. Preferably, the incorporation of the nucleotide building blocks into the elongated nucleic acid molecule is associated with a detectable increase in the fluorescence, preferably with a transient increase in the fluorescence. For example, nucleotide building blocks may be used which carry a fluorescent labelling group on the portion of the molecule which is cleaved off when the building block is incorporated into the primer, e.g., on the γ-phosphate group.

When a nucleotide building block is cleaved off from the synthesized nucleic acid molecule, a time-dependent change of fluorescence may be determined due to the interaction of fluorescent labelling groups incorporated in nucleic acid strands with neighbouring groups, for example with chemical groups of the nucleic acids, in particular nucleobases such as, for example, G, or/and neighbouring fluorescent labelling groups, and these interactions leading to a change in fluorescence, in particular in fluorescence intensity, compared to the fluorescent labelling groups in "isolated" form, owing to quenching processes or/and energy transfer processes. The removal by cleavage of individual nucleotide building blocks alters the overall fluorescence, for example the fluorescence intensity of an immobilized nucleic acid strand, and this change is a function of the removal by cleavage of individual nucleotide building blocks, i.e., a function of time.

In certain embodiments, association of a labelled nucleotide with the biomolecule complex is detected by measuring polarisation of the emitted photons. The polarisation of excited states' photons is changed by the rotational movement of the light emitting nucleotide labels and can be used for identifying free moving contra bound labelled nucleotides in the polymerisation process.

This time-dependent change in fluorescence during elongation and/or degradation may be recorded in parallel for a multiplicity of nucleic acid molecules and correlated with the base sequence of the individual nucleic acid strands. Preference is given to using those fluorescent labelling groups which, when incorporated in the nucleic acid strand, are, at least partially, quenched so that the fluorescence intensity is increased after the nucleotide building block containing the labelling group or a neighbouring building block causing quenching has been removed by cleavage.

During incorporation and/or removal of individual nucleotide building blocks, it is possible to measure a change in fluorescence intensity of the nucleic acid strand or/and the incorporated or cleaved-off nucleotide building block, owing to quenching processes or energy transfer processes. This change in fluorescence intensity with time depends on the base sequence of the nucleic acid strand studied and can therefore be correlated with the sequence.

The complete sequence of the nucleic acid molecule may be determined by using a mixture of nucleotide building blocks, labelled on all four different bases, for example on A, G, C and T, or on combinations of two or three different bases. It is possible, where appropriate, to attach to the nucleic acid strand to be studied also a "sequence identifier", i.e., a labelled nucleic acid of known sequence, for example by enzymatic reaction using ligase or/and terminal transferase, so that at the start of sequencing initially a known fluorescence pattern and only thereafter the fluorescence pattern corresponding to the unknown sequence to be studied is obtained.

The detection comprises irradiating light into the support, preferably by means of a laser, or by another suitable light source, in order to cause excitation of the fluorescent labelling groups. It is possible, in this connection, to use one or more laser beams, for example an expanded laser beam, having a cross section of approx. 1-20 mm, and/or multiple laser beams. The detection preferably comprises a multipoint fluorescence excitation by lasers, for example a dot matrix of laser dots generated via diffraction optics (cf. WO 2002/097406) or a quantum well laser.

Fluorescence emission of a plurality of nucleic acid strands may be detected in parallel using a detector matrix which comprises, for example, an electronic detector matrix, for example a CCD camera, a CMOS detector matrix, e.g., a CMOS camera, or an avalanche photodiode matrix. The detection may be carried out in such a way that fluorescence excitation and detection are carried out in parallel on a part or all nucleic acid strands studied. Preference is given to carrying out the detection on fluorescence light which is emitted essentially orthogonally from the support surface through the reaction space or through the support body.

The detection may be carried out, for example, by means of a single molecule detection, for example by fluorescence correlation spectroscopy, which involves exposing a very small, preferably confocal, volume element, for example from 10⁻²¹ to 10⁻¹⁰ I, to the excitation light of a laser, or another suitable light source, which light excites the receptors present in this measuring volume so that the latter emit fluorescence light, the fluorescence light emitted from said measuring volume being measured by means of a photodetector and the change in the measured emission with time being correlated with the concentration of the analyte, so that it is possible to identify, at an appropriately high dilution, individual molecules in said measuring volume. Details of the procedure and of the apparatus used for detection can be found in the disclosure of EP 0 679 251, the content of which is herein incorporated by reference. The confocal determination of single molecules is furthermore described in Rigler and Mets (Soc. Photo-Opt. Instrum. Eng. 1921 (1993), 239 ff.) and Mets and Rigler (J. Fluoresc. 4 (1994) 259-264), the contents of which are herein incorporated by reference.

Alternatively, or additionally, detection may also be carried out by way of time-resolved decay measurement, called "time gating", as described, for example, by Rigler et al., "Picosecond Single Photon Fluorescence Spectroscopy of Nucleic Acids", in: "Ultrafast Phenomena", D.H. Auston, Ed., Springer 1984, the content of which is herein incorporated by reference. Here, the fluorescent molecules are excited in a measuring volume followed by, e.g., at a time interval of ≥ 100 ps, opening a detection interval on the photodetector. In this way it is possible to keep background signals generated by Raman effects sufficiently low so as to enable single molecules to be detected in an essentially interference-free manner.

The second aspect of the present disclosure relates to a method of cleaning a previously used support wherein said support is adapted for use in devices and methods for analyzing an event, e.g., a single molecule event. If not indicated differently, the features of the first aspect also applies to the second aspect.

A previously used support as understood herein encompasses a support on which a previous analysis, e.g., single molecule analysis has been performed. Thus, the support has attached a biological moiety such as a biomolecule to the at least one sample spot and is optionally contaminated with further components, e.g., components of the sample on which the analysis has been performed. By the method as disclosed herein, the biological moiety, e.g., the biomolecule attached to the sample spot and optional further components are removed from the support without causing substantial damage.

An embodiment of the second aspect relates to a method of cleaning a previously used support comprising:
(i) providing a support comprising a substrate and at least one sample spot on the surface of the support, wherein a biological moiety, e.g., a biomolecule is attached to the at least one sample spot.
(ii) subjecting the support from step (i) to a treatment with an alkaline solution comprising e.g., an alkaline phosphate such as potassium phosphate and optionally a surfactant and/or a chelator, wherein the treatment is performed at an elevated temperature, particularly under boiling;
(iii) optionally rinsing the support after step (ii) e.g., with water;
(iv) subjecting the support after step (ii) or (iii) to a treatment with an acidic/oxidative solution comprising a strong inorganic acid, e.g, sulfuric acid and a peroxide, e.g., hydrogen peroxide;
(v) optionally rinsing the support after step (iv) e.g., with water and/or an anhydrous organic solvent, e.g., ethanol;
(vi) optionally drying the support after step (iv) or (v) with an inert gas, e.g., Ar, N₂ or any mixture thereof;
(vii) subjecting the support after step (iv), (v) or (vi) to a plasma treatment, particularly to an O₂ plasma treatment.

In certain embodiments of this aspect, the support to be cleaned comprises a substrate and at least one sample spot on the surface of the support, wherein the substrate is at least partially coated with a layer of diamond-like and/or amorphous carbon and wherein the layer of diamond-like and/or amorphous carbon does not extend over the at least one binding spot.

In further embodiments of this aspect, the support to be cleaned comprises a substrate and at least one sample spot on the surface of the support, wherein the substrate is at least partially coated with a layer of an organic passivating material, e.g., a poly(ethyleneoxy) group containing passivating material, and wherein the layer of organic passivating material does not extend over the at least one sample spot.

In certain embodiments of this aspect, a biological moiety such as a biomolecule is attached to the cleaned support after step (vii). Further, the support surface surrounding the sample spots may be regenerated, e.g., treated with passivating reagent that inhibits adhesion of biomolecules such as proteins, in order to inhibit adhesion of biomolecules and/or other sample constituents. In certain embodiments, passivating the substrate may comprise a treatment with a PEGylating reagent, such as an alkoxy PEG silane, e.g., a methoxy PEG silane. Alternatively, regenerating may comprise coating the surface of said support with a fresh layer of diamond-like and/or amorphous carbon. Thereafter, the support is ready for use in a new analysis of an event, e.g., a single molecule event. The regenerating step is typically performed before the step of attaching a biological moiety, e.g., a biomolecule. When using a support coated with a carbon film as described above in the first aspect, the regenerating step is not necessary in certain embodiments.

In some embodiments, the attachment of a biological moiety, e.g., a biomolecule comprises:
(viii) activating the surface of the at least one sample spot, e.g., subjecting the support after step (vii) to a treatment with a thiol reagent;
(ix) optionally rinsing the support after step (viii) e.g., with an organic solvent, such as anhydrous ethanol;
(x) optionally drying the support after step (viii) or (ix) with an inert gas, e.g., Ar, N₂ or any mixture thereof;
(xi)optionally regenerating the surface of said support, e.g., passivating the substrate, e.g., by treatment with a PEGylating reagent, such as an alkoxy PEG silane, e.g., a methoxy PEG silane, or by coating the surface of said support with a layer of diamond-like and/or amorphous carbon;
(xii) optionally rinsing the support after step (xi) e.g., with an organic solvent, e.g., acetone and/or anhydrous ethanol;
(xiii) optionally drying the support after step (xi) or (xii) with an inert gas, e.g., Ar, N₂ or any mixture thereof; and
(xiv) binding a biomolecule to the activated surface of the at least one sample spot, wherein the support is ready for use in a new analysis of a single molecule event.

Further, the present disclosure is explained in detail by reference to the following specific embodiments.

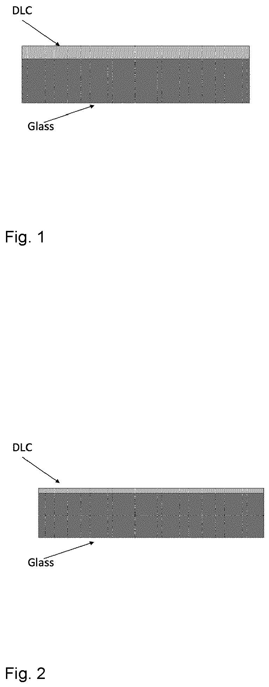

Embodiment 1 of Fig. 1 (PRIOR ART) shows an optically transparent substrate (e.g., quartz; fused silica; or glass) coated with a layer of diamond-like carbon (DLC) deposited by chemical vapor deposition (CVD) with a thickness ranging from about 1 nm - 10 µm. Following deposition, the DLC film is exposed to a fluorine-containing plasma.

Embodiment 2 of Fig. 2 (PRIOR ART) shows an optically transparent substrate (e.g., quartz; fused silica; or glass) coated with a layer of (DLC) or amorphous carbon deposited by Atomic Layer Deposition (ALD) with a thickness ranging from about 1 nm - 100 nm. Following deposition, the film is exposed to a fluorine-containing plasma.

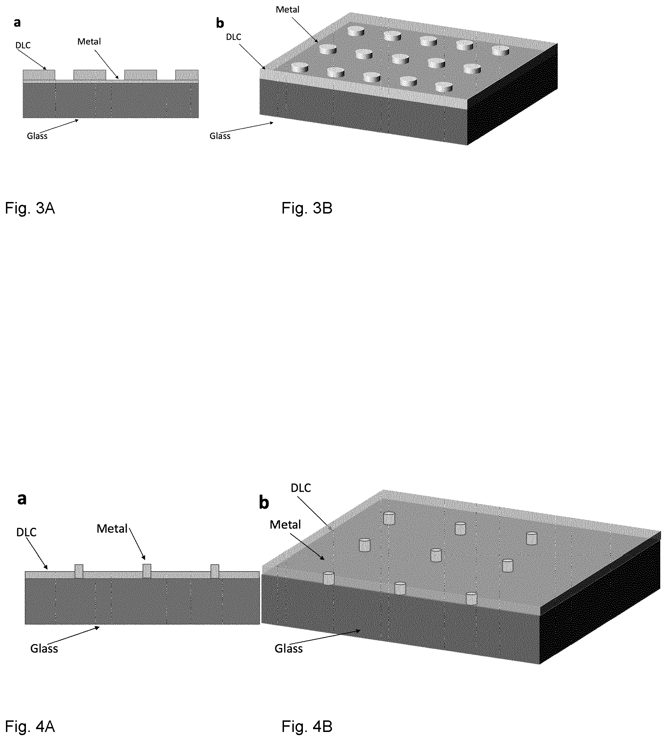

Embodiments 3 and 4 of Fig. 3A and 3B (PRESENT DISCLOSURE) show an optically transparent substrate (e.g., quartz; fused silica; or glass) coated with DLC, deposited by CVD or ALD, with a thickness ranging from 1 nm - 10 µm. Following deposition, the DLC is coated with a photoresist, optionally after treatment with oxygen plasma. Then, the photoresist is patterned by a lithographic technique (e.g., optical or electron beam lithography, or any other suitable technique) and developed to form a stencil in the resist according to the desired pattern.

According to Fig. 3A, an underlying metal or metal oxide layer is present. Openings are formed in the carbon film by etching to expose selected areas of the underlying film. These openings may be form sample spots. The carbon film is exposed to a fluorine-containing plasma.

According to Fig. 3B, metal or metal oxide features are created on top of the carbon film, e.g., by a conventional liftoff patterning. The carbon film is exposed to a fluorine-containing plasma.

Embodiment 5 of Fig. 4A and 4B (PRESENT DISCLOSURE) shows an optically transparent substrate (e.g., quartz; fused silica; or glass) patterned with metal spots using lithographic patterning processes, such that a pattern of metal spots is arrayed at predetermined positions on the surface of the substrate. The metal is a non-adherent material, i.e., it is unreactive with DLC (or in the case of ALD, with the ALD linker). Au, Cu and Ni are examples of such metals [7]. The surface is then selectively coated with DLC. Because the DLC does not bind to the metal, the carbon will not deposit on the metal, but only on the glass. The support is exposed to a fluorine-containing plasma.

### References

1. Bernhard, C., et al., Repelling and ordering: the influence of poly(ethylene glycol) on protein adsorption. Physical Chemistry Chemical Physics, 2017. 19(41): p. 28182-28188.
2. Pan, H., et al., A simple procedure to improve the surface passivation for single molecule fluorescence studies. Physical Biology, 2015. 12(4): p. 045006.
3. Park, S.R., et al., A Single-Molecule Surface-Based Platform to Detect the Assembly and Function of the Human RNA Polymerase II Transcription Machinery. Structure, 2020. 28(12): p. 1337-1343.e4.
4. Wong, I. and C.-M. Ho, Surface molecular property modifications for poly(dimethylsiloxane) (PDMS) based microfluidic devices. Microfluidics and Nanofluidics, 2009. 7(3): p. 291.
5. Yonezawa, K., et al., Evaluation of Antibacterial and Cytotoxic Properties of a Fluorinated Diamond-Like Carbon Coating for the Development of Antibacterial Medical Implants. Antibiotics (Basel), 2020. 9(8).
6. Horikawa, A.M., Shunto; Hasebe, Terumitsu; Matsumoto, Tomohiro; Tanaka, Minoru; akahashi, Koki; Suzuki, Tetsuya, Fluorine-incorporated amorphous carbon coating inhibits adhesion of blood cells to biomaterials. Sensors and Materials, 2017. 29(6): p. 9.
7. Outka, D.A.H., Wen L.; Boehme, D. R.; Yang, N. Y. C.; Ottesen, D. K.; Johnsen, H. A., Compilation of diamond-like carbon properties for barriers and hard coatings, in Sandia Report. 1994.
8. Rajak, D.K., et al., Diamond-Like Carbon (DLC) Coatings: Classification, Properties, and Applications. Applied Sciences, 2021. 11(10): p. 4445.
9. Robertson, J., Diamond-like amorphous carbon. Materials Science and Engineering: R: Reports, 2002. 37(4): p. 129-281.
10. Bendavid, A., et al., The properties of fluorine-containing diamond-like carbon films prepared by pulsed DC plasma-activated chemical vapour deposition. Diamond and Related Materials, 2010. 19(12): p. 1466-1471.
11. Schvartzman, M., et al., Plasma fluorination of carbon-based materials for imprint and molding lithographic applications. Applied Physics Letters, 2008. 93(15): p. 3.
12. Schvartzman, M., et al., Fluorinated diamondlike carbon templates for high resolution nanoimprint lithography. Journal of Vacuum Science & Technology B, 2008. 26(6): p. 2394-2398.
13. Carvalho, I., et al., Overview on the Antimicrobial Activity and Biocompatibility of Sputtered Carbon-Based Coatings. Processes, 2021. 9(8): p. 1428.
14. Hasebe, T., et al., Fluorine doping into diamond-like carbon coatings inhibits protein adsorption and platelet activation. Journal of Biomedical Materials Research Part A, 2007. 83A(4): p. 1192-1199.

## Claims

1. A support comprising a substrate and at least one sample spot on the surface of the support,
wherein the substrate is at least partially coated with a layer of diamond-like and/or amorphous carbon and wherein the layer of diamond-like and/or amorphous carbon does not extend over the at least one binding spot.

2. The support of claim 1, which is at least substantially planar.

3. The support of any one of the preceding claims, which comprises a plurality of sample spots.

4. The support of any one of the preceding claims, wherein the at least one sample spot comprises at least one metal or metal oxide.

5. The support of any one of the preceding claims, wherein the at least one sample spot has a diameter of about 1 nm to about 100 µm, particularly a diameter of about 2 nm to about 50 µm.

6. The support of any one of the preceding claims,
(i) wherein the at least one sample spot has an upper face distal to the substrate and wherein the upper face of is of even elevation with the surrounding layer of diamond-like and/or amorphous carbon;
(ii) wherein the at least one sample spot has an upper face distal to the substrate and wherein the upper face of the sample spot is above the surrounding layer of diamond-like and/or amorphous carbon; or
(iii) wherein the at least one sample spot has an upper face distal to the substrate and wherein the upper face of the sample spot is below the surrounding layer of diamond-like and/or amorphous carbon.

7. The support of any one of the preceding claims,
(i) wherein the at least one sample spot extends through the carbon layer such that its lower face is in direct contact with the surface of the substrate, or
(ii) wherein the at least one sample spot does not extend through the carbon layer such that its lower face is in direct contact with the surface of the carbon layer, or
(iii) wherein the at least one sample spot is located upon a pillar that has been etched into a planar substrate, wherein the layer of diamond-like and/or amorphous carbon coats the sidewalls of the pillar as well as the planar surface of the substrate.

8. The support of any one of the preceding claims, wherein the layer of diamond-like and/or amorphous carbon has a thickness of about 0.3 nm to about 200 µm, particularly about 10 nm to about 100 µm, and more particularly about 1 µm to about 50 µm.

9. The support of any one of the preceding claims, wherein the layer of diamond-like and/or amorphous carbon is a fluorinated diamond-like and/or fluorinated amorphous carbon.

10. The support of any one of the preceding claims, wherein a biological moiety, particularly a single biomolecule is attached to at least one of the sample spots, wherein the biological moiety is particularly a nucleic acid-polymerizing enzyme, e.g., an RNA polymerase or a DNA-polymerase.

11. Use of a support of any one of claims 1-10 for analyzing an event, wherein said event is associated with emission of electromagnetic radiation from said sample spot, particularly a single molecule event comprising nucleic acid sequence determination, occurring on the at least one sample spot, particularly for separately analyzing a plurality of single molecule events each occurring on at least one sample spot, and more particularly for separately analyzing a plurality of single molecule events is analyzed in parallel.

12. A method for analyzing an event comprising:
(i) providing a support of any one of claims 1-10,
(ii) immobilizing a biological moiety on the at least one sample spot of the support, and
(iii) analyzing an event associated with said biological moiety by detecting electromagnetic radiation from said sample spot.

13. The method of claim 12, wherein the event comprises a sequence analysis of a single nucleic acid molecule.

14. A device for analyzing an event comprising:
(iv) a support of any one of claims 1-10,
(v) means for illuminating the at least one sample spot on the support with radiation, and
(vi) means for analyzing an event on said the at least one sample spot by detecting electromagnetic radiation from said spot.

15. The device of claim 14 adapted for the sequence analysis of a single nucleic acid molecule.
